(19) Europäisches Patentamt
European Patent Office
Office européen des brevets

(11) EP 1 219 608 A1

(12) EUROPEAN PATENT APPLICATION
published in accordance with Art. 158(3) EPC

(43) Date of publication:
03.07.2002 Bulletin 2002/27

(21) Application number: 00962848.8

(22) Date of filing: 26.09.2000

(51) Int Cl.[7]: C07D 239/36, C07D 405/06, C07D 413/12, A61K 31/505, A61K 31/506, A61P 11/00, A61P 9/00, A61P 1/02

(86) International application number:
PCT/JP00/06601

(87) International publication number:
WO 01/23361 (05.04.2001 Gazette 2001/14)

(84) Designated Contracting States:
AT BE CH CY DE DK ES FI FR GB GR IE IT LI LU MC NL PT SE
Designated Extension States:
AL LT LV MK RO SI

(30) Priority: 27.09.1999 JP 27184399

(71) Applicant: ONO PHARMACEUTICAL CO., LTD.
Osaka-shi, Osaka 541-8526 (JP)

(72) Inventors:
- KOJIMA, Tsutomu, Fukui Research Institute
Mikuni-cho, Sakai-gun, Fukui 913-0032 (JP)
- OKADA, Takanori, Fukui Research Institute
Mikuni-cho, Sakai-gun, Fukui 913-0032 (JP)
- HACHIYA, Katsutoshi, Fukui Research Institute
Mikuni-cho, Sakai-gun, Fukui 913-0032 (JP)
- MOTOI, Takahiro, Fukui Research Institute
Mikuni-cho, Sakai-gun, Fukui 913-0032 (JP)
- HASHIMOTO, Shinsuke,
Fukui Research Institute
Mikuni-cho, Sakai-gun, Fukui 913-0032 (JP)

(74) Representative: Henkel, Feiler, Hänzel
Möhlstrasse 37
81675 München (DE)

# (54) PYRIMIDINE DERIVATIVES, PROCESS FOR PREPARING THE DERIVATIVES AND DRUGS CONTAINING THE SAME AS THE ACTIVE INGREDIENT

(57) A pyrimidine compound of formula (I), wherein $R^1$ is H, F; $R^2$ is $-CH(OR^3)_2$, t-butoxycarbonyl, $-CH=CH_2$, furan-2-yl, -CHO, -COOH or a group of formula (II); $R^3$ is methyl or ethyl; $R^4$ is methyl, isopropyl, n-butyl, t-butyl, phenyl, 1-methylcyclopropyl, benzyl, 3-methylbenzyl, 3-trifluoromethylbenzyl, 3,4-methylenedioxybenzyl, α,α-dimethylbenzyl, α,α-dimetyl-3-methylbenzyl, α,α-dimetyl-3-trifluoromethylbenzyl or α,α-dimetyl-3,4-methylenedioxybenzyl; or a non-toxic salt thereof.

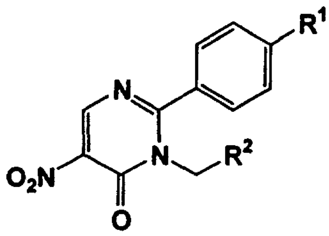
(I) ;

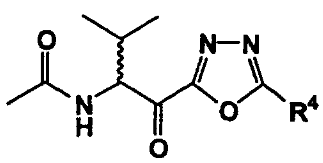
(II)

A pyrimidine compound of formula (I) or a non-toxic salt thereof is useful as an important intermediate of pharmaceutical compound and/or a pharmaceutical, especially elastase inhibitor.

EP 1 219 608 A1

## Description

Technical Field

This invention relates to a novel pyrimidine compound. More particularly, this invention relates to:

(1) a novel pyrimidine compound of formula (I)

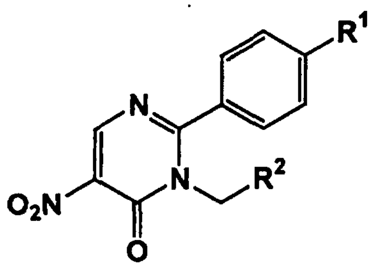

(I)

wherein all the symbols are as hereinafter defined;
or a non-toxic salt thereof, which is useful as an intermediate of a pharmaceutical compound or a pharmaceutical,
(2) a process for the preparation thereof,
(3) a pharmaceutical composition comprising the compound of formula (I) as active ingredient and
(4) a process for the preparation of pyrimidine compound of formula (E)

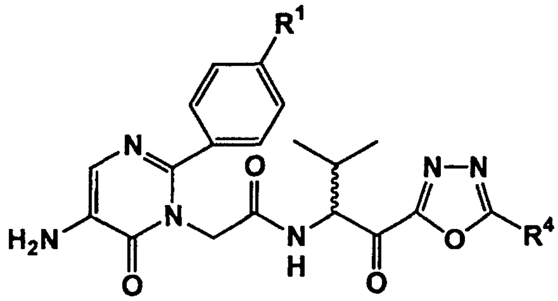

(E)

wherein all the symbols are as hereinafter defined;
or a non-toxic salt thereof, which is useful as a pharmaceutical compound; using the compound of formula (I).

Background

Pyrimidine compounds that are useful for pharmaceutical, were disclosed in:

(1) EP 528633 (JP-A-5-286946), WO 93/21210 (JP-A-7-505876), WO 93/21214 (JP-A-7-505877) and J. Med. Chem., 38, 98 (1995), as elastase inhibitors,
(2) WO 98/24806, as serine protease inhibitors (especially, elastase inhibitors),
(3) WO 96/33974 and WO 98/09949, as chymase inhibitors,
(4) WO 95/26958 (JP-A-9-511249) and J. Med. Chem., 39, 2438 (1996), as interleukin-1β converting enzyme inhibitors.

In description of WO 98/24806, the pyrimidine compound of formula (E) can be prepared by 8 steps from a reaction of amidine compound of formula (X-1) in which $R^{2X}$ is dimethoxymethyl or diethoxymethyl, and diethylethoxymethylene malonate of formula (Y), or by 9 steps from a reaction of amidine compound of formula (X-1) in which $R^{2X}$ is vinyl, and diethylethoxymethylene malonate of formula (Y). From the above, it is desired that the compound of formula (E) can be prepared at a commercially large-scale synthesis by more efficient and in fewer step.
Processes for the preparation of various compounds going through the compound of formula (X)

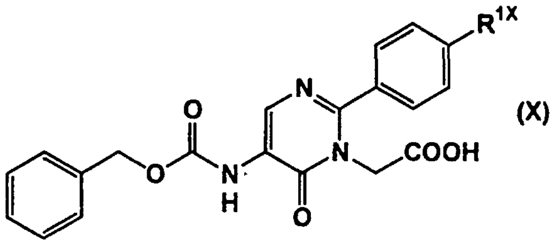

(X)

wherein $R^{1X}$ is a various substituent;
as an intermediate, were shown in the specifications of above (1)-(4).

Accordingly, the compound of formula (X) is a very important compound as an intermediate for pharmaceuticals.

The process for the preparation of the compound of formula (X) was depicted as following scheme 1.

In scheme 1,

$R^{1X}$ are various substituents,
$R^{2X}$ is dimethoxymethyl, diethoxymethyl or vinyl,
$Et_3N$ is triethylamine,
DPPA is diphenylphosphryl azide,
BnOH is benzyl alcohol,
Cbz is benzyloxycarbonyl.

Scheme 1

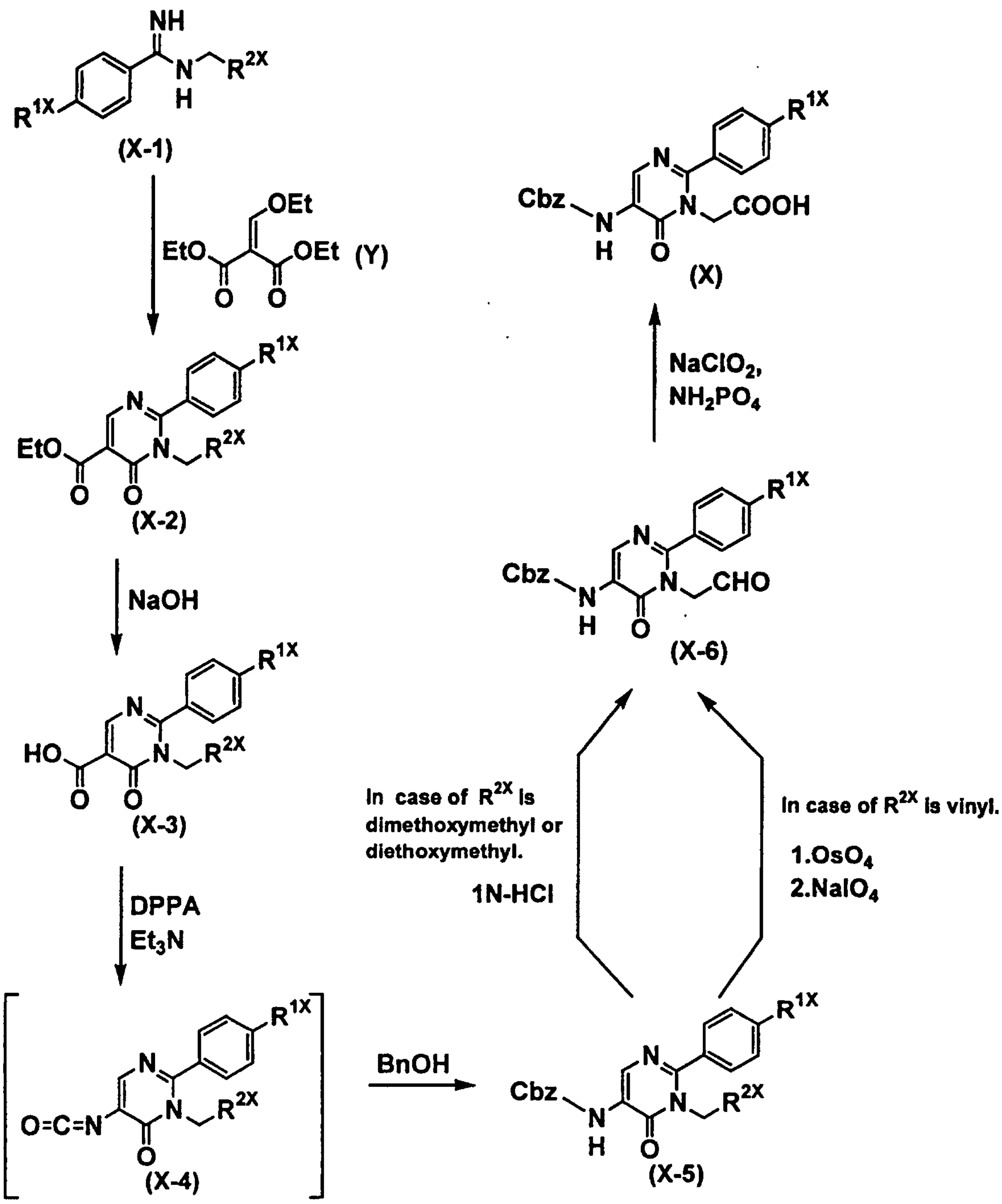


In the conventional method of scheme 1, a compound of formula (X-5) can be prepared by subjecting a compound of formula (X-3) to Curtius rearrangement reaction. In the rearrangement reaction, a large amount of nitrogen gas explosively was produced. Therefor, notwithstanding that there was no danger of explosion in small-scale synthesis, there was danger in a commercially large-scale synthesis.

Moreover, in description of WO 98/24806, a pyrimidine compound of formula (E)

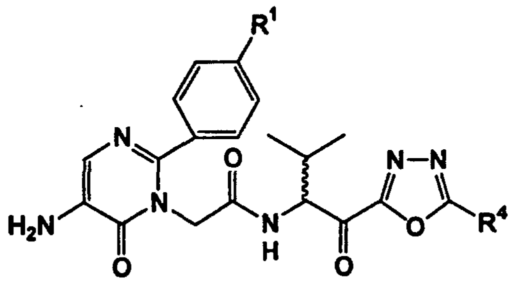

(E)

wherein $R^1$ is hydrogen or fluorine,
$R^4$ is methyl, isopropyl, n-butyl, t-butyl, phenyl, 1-methylcyclopropyl, benzyl, 3-methylbenzyl, 3-trifluoromethylbenzyl, 3,4-methylenedioxybenzyl, α,α-dimethylbenzyl, α,α-dimetyl-3-methylbenzyl, α,α-dimetyl-3-trifluoromethylbenzyl or α,α-dimetyl-3,4-methylenedioxybenzyl;
or a non-toxic salt thereof was disclosed to be useful as serine protease inhibitors, especially, elastase inhibitors.

A process for the preparation thereof was depicted as following scheme 2 using a compound of formula (E-1) as a starting material, which is a compound of formula (X) in which $R^{1X}$ is hydrogen or fluorine.

In scheme 2,

$R^1$ and $R^4$ are as hereinbefore defined,
EDC is 1-ethyl-3-[3-(dimethylamino)propyl]carbodiimide,
HOBT is 1-hydroxybenzotriazole,
NMM is N-methylmorpholine,
DMF is dimethylformamide.

**Scheme 2**

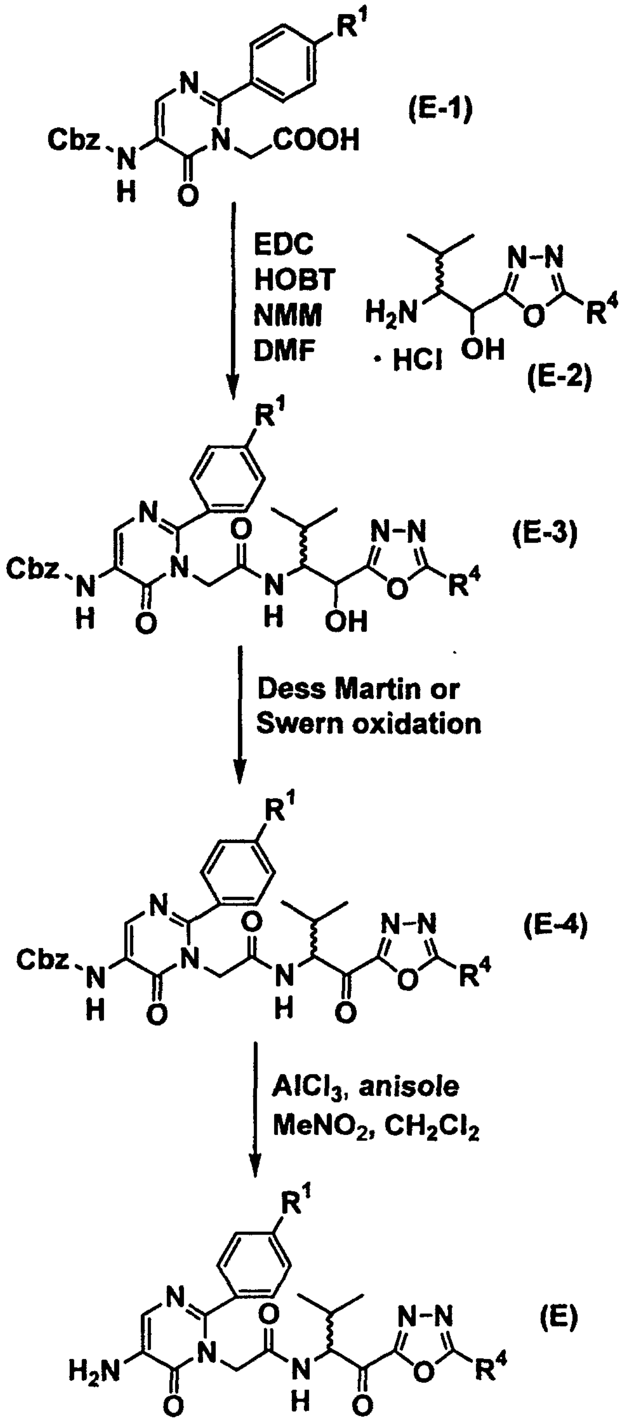


In description of WO 98/24806, the pyrimidine compound of formula (E) can be prepared by 8 steps from a reaction of amidine compound of formula (X-1) in which $R^{2X}$ is dimethoxymethyl or diethoxymethyl, and diethylethoxymethylene malonate of formula (Y), or by 9 steps from a reaction of amidine compound of formula (X-1) in which $R^{2X}$ is vinyl, and diethylethoxymethylene malonate of formula (Y). From the above, it is desired that the compound of formula (E) can be prepared at a commercially large-scale synthesis by more efficient and in fewer step.

Disclosure of the invention

Energetic investigations have been carried out in order to accomplish the above purpose. The present inventors have found a process of the following scheme 3.

In scheme 3,

$R^1$ is hydrogen or fluorine,
$R^{2-A}$ is $-CH(OR^3)_2$, t-butoxycarbonyl, $-CH=CH_2$ or furan-2-yl,
$R^3$ is methyl or ethyl,
$R^{10}$ is methyl or ethyl,
$R^4$ is methyl, isopropyl, n-butyl, t-butyl, phenyl, 1-methylcyclopropyl, benzyl, 3-methylbenzyl, 3-trifluoromethylbenzyl, 3,4-methylenedioxybenzyl, α, α-dimethylbenzyl, α, α-dimetyl-3-methylbenzyl, α, α-dimetyl-3-trifluoromethylbenzyl or α, α-dimetyl-3,4-methylenedioxybenzyl.

## Scheme 3

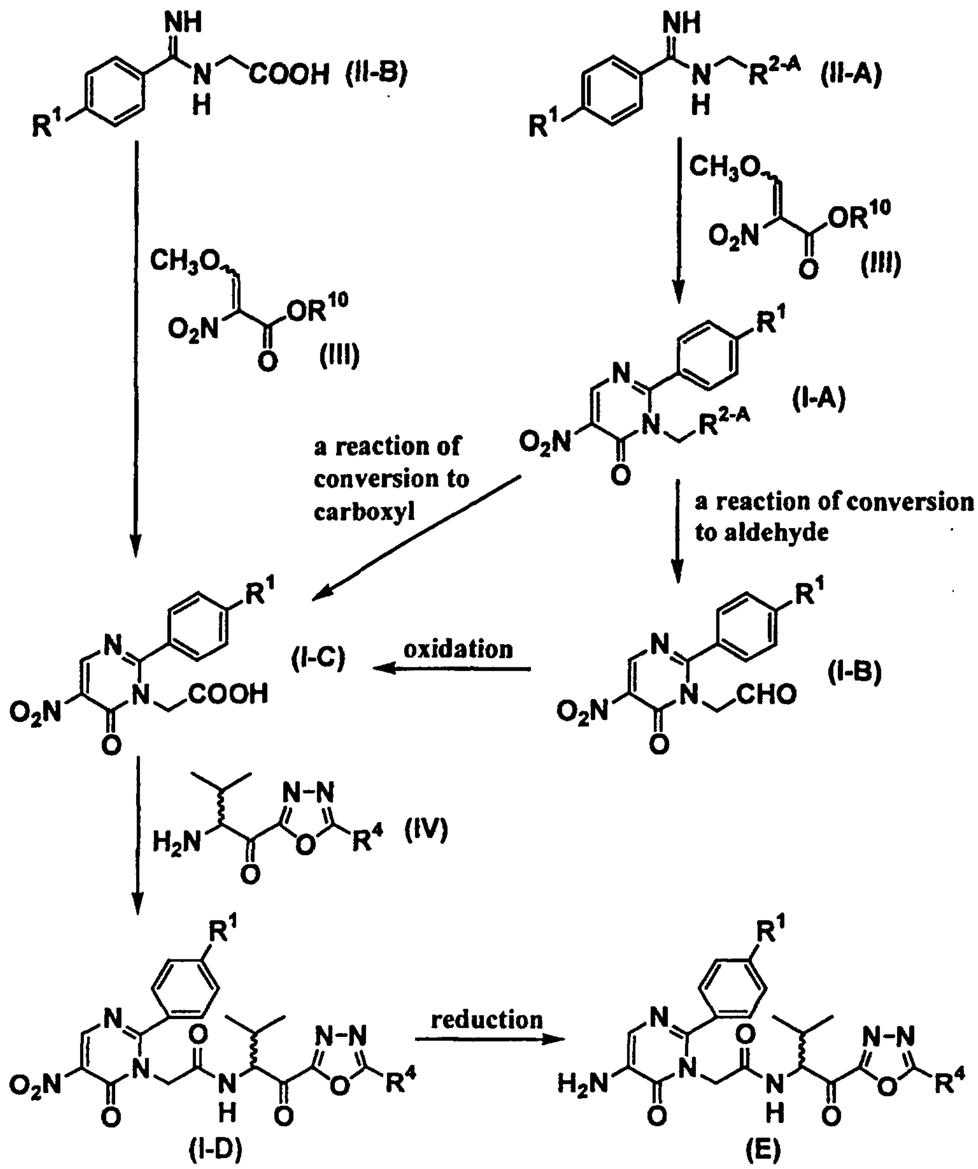


According to a process shown in scheme 3, a compound of formula (E) can be prepared without Curtius rearrangement reaction.

Besides, a compound of formula (E) could be prepared in 3-5 steps from the reaction of an amidine compound of formula (II-A) or (II-B) and 3-methoxy-2-nitroacrylate compound of formula (III).

Compounds of formula (I-A), (I-B), (I-C) and (I-D) were novel compounds, and important intermediate compounds of formula (E), which is serine protease inhibitors, especially, elastase inhibitors.

Besides, the present inventors found that a compound of formula (I-D) has elastase inhibitory effect.

The reaction of phenylamidine which has no substituents on nitrogen atom and 3-ethoxy-2-nitroacrylic acid methyl ester was known [J. Heterocyclic Chem., 22, 337 (1985)]. However, the present reaction of an amidine compound of formula (II-A) and (II-B) which has a substituent on nitrogen atom as a starting material, and 3-methoxyl-2-nitroacrylate compound was entirely novel.

The above document disclosed that 5-nitro-6-oxo-1,6-dihydropyrimidine compound could be prepared at most

in moderate yield by the reaction of 1 equivalent of 3-ethoxy-2-nitroacrylic acid methyl ester with 3 equivalents of an amidine compound. For example, 1 equivalent of 3-ethoxy-2-nitroacrylic acid methyl ester was added to a solution of 3 equivalents of phenylamidine and after the reaction, 2-phenyl-5-nitro-6-oxo-1,6-diydropyrimidine was obtained at 59% yield.

On the other hand, in the example of the present invention, 1 equivalent of 3-ethoxy-2-nitroacrylate was dropped slowly into a solution of 1.0-1.1 equivalents of amidine compound in the presence or absence of an additional compound. Hereby, synthesis of side products was depressed and 2-phenyl-5-nitro-6-oxo-1,6-diydropyrimidine compound was obtained in high-yield.

This invention relates to:

(1) a novel pyrimidine compound of formula (I):

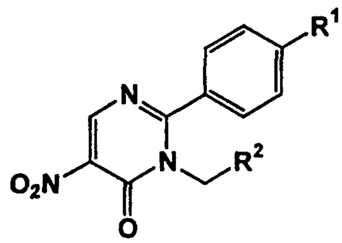

(I)

wherein $R^1$ is hydrogen or fluorine,
$R^2$ is $-CH(OR^3)_2$, t-butoxycarbonyl, $-CH=CH_2$ or furan-2-yl, -CHO, -COOH or

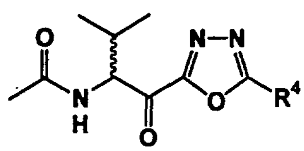

$R^3$ is methyl or ethyl,
$R^4$ is methyl, isopropyl, n-butyl, t-butyl, phenyl, 1-methylcyclopropyl, benzyl, 3-methylbenzyl, 3-trifluoromethylbenzyl, 3,4-methylenedioxybenzyl, α,α-dimethylbenzyl, α,α-dimetyl-3-methylbenzyl, α,α-dimetyl-3-trifluoromethylbenzyl or α,α-dimetyl-3,4-methylenedioxybenzyl;
or a non-toxic salt thereof, which is useful as an intermediate of pharmaceutical compound or a pharmaceutical,
(2) a process for the preparation thereof,
(3) a pharmaceutical composition comprising the compound of formula (I) as active ingredient and
(4) a process for the preparation of pyrimidine compound of formula (E):

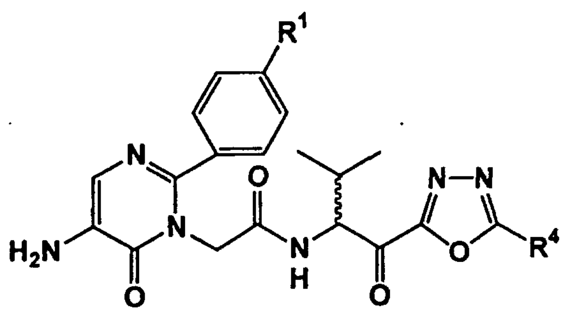

(E)

wherein all the symbols are as hereinbefore defined;
or a non-toxic salt thereof, which is useful as a pharmaceutical compound; using the compound of formula (I).

In the invention, as is apparent to one skilled in the art, unless otherwise indicated, the mark:

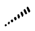

shows that the bond is on the other side of paper (α-configuration), the mark:

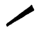

shows that the bond is in front of paper (β-configuration), the mark:

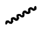

shows that the bond are α-, β-configuration or a mixture thereof, or E-, Z-configuration a mixture thereof, the mark:

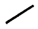

shows that the bond is a mixture of α- configuration and β- configuration.

Unless otherwise specified, all isomers are included in the present invention. For example, alkyl, alkoxy and alkylene include straight and branched isomers. Isomers based on double bond, ring, fused ring (E, Z, cis, trans), isomers resulting from the presence of asymmetric carbon(s) (R-configuration, S-configuration, α-configuration, β-configuration, enantiomers, diastereoisomers), optically active compounds having optical rotation (D, L, d, l-configuration), polar compounds obtained by chromatographic separations (highly polar compound, less polar compound), equilibrium compounds, the mixtures are existed by free ratio, racemic mixtures are included in the present invention.

Process for the Preparation

The compounds of the present invention of formula (I), can be prepared by the following methods or the methods described in Examples.

[1] The compound in which $R^2$ is $-CH(OR^3)_2$, t-butoxycarbonyl, $-CH=CH_2$ or furan-2-yl in the compound of the present invention of formula (I), that is the compound of formula (I-A):

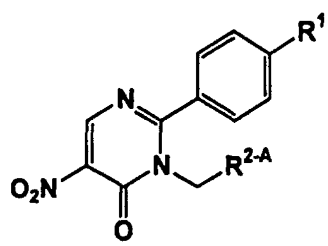

(I-A)

wherein $R^{2\text{-}A}$ is $-CH(OR^3)_2$, t-butoxycarbonyl, $-CH=CH_2$ or furan-2-yl, and the other symbols are as hereinbefore defined;
may be prepared by reacting a compound of formula (II-A):

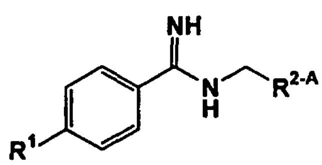

(II-A)

wherein all the symbols are as hereinbefore defined;
with 3-methoxy-2-nitroacrylate compound of formula (III):

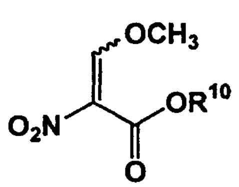

(III)

wherein $R^{10}$ is methyl or ethyl.

As previously noted, the above reaction is novel, for example, may be carried out in an organic solvent (e.g. methanol, benzene, toluene, dimethylformamide), in the presence or absence of an additional compound (e.g. calcium acetate, manganese acetate, cesium fluoride, calcium chloride, magnesium chloride or a mixture thereof), at -20-150° C.

[2] The compound in which $R^2$ is -CHO in the compounds of the present invention of formula (I), that is the compound of formula (I-B):

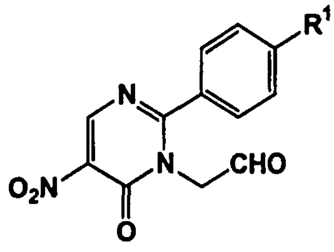

(I-B)

wherein all the symbols are as hereinbefore defined;
may be prepared by following methods (a) or (b).

(a) The compound of formula (I-B) may be prepared by subjecting the above compound of formula (I-A) in which $R^{2-A}$ is $-CH(OR^3)_2$, that is the compound of formula (I-A-a):

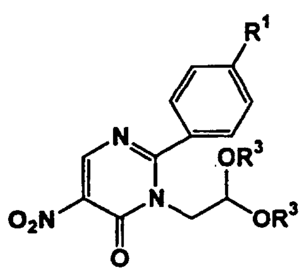

(I-A-a)

wherein all the symbols are as hereinbefore defined;
to deprotection under acidic conditions.

The reaction of deprotection under acidic conditions is known, for example, it may be carried out in an organic solvent (e.g. methylene chloride, chloroform, dioxane, ethyl acetate, anisole), water or without a solvent, using an organic acid (e.g. acetic acid, trifluoroacetic acid, methanesulfonic acid, trimethylsilyl iodide), or an inorganic acid (e.g. hydrochloric acid, sulfuric acid) or a mixture thereof (e.g. hydrogen bromide acetic acid), at 0-100°C.

(b) The compound of formula (I-B) may be prepared by subjecting the above compound of formula (I-A) in which $R^{2-A}$ is $-CH=CH_2$, that is the compound of formula (I-A-b):

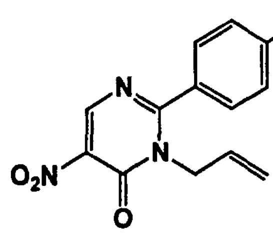

(I-A-b)

wherein all the symbols are as hereinbefore defined;
to oxidative cleavage.

The reaction of oxidative cleavage is known, for example, it may be carried out in an organic solvent (e.g. methylene chloride, chloroform, methanol, ethanol, t-butanol) or a mixture thereof, using a reagent for oxidative cleavage (e.g. ozone, potassium permanganate), at -78-50°C.

As will be apparent to those skilled in the art, reagents for oxidative cleavage other than the above reagents are also preferred. For example, reagents described in Comprehensive Organic Transformations (Richard C. Larock, VCH Publishers, Inc., (1989) page 595 - 596), may be used.

[3] The compound in which $R^2$ is -COOH in the compounds of the present invention of formula (I), that is the compound of formula (I-C):

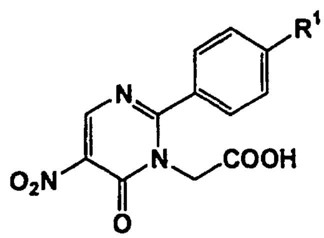

(I-C)

wherein all the symbols are as hereinbefore defined;
may be prepared by following methods (c) - (g).

(c) The compound of formula (I-C) may be prepared by subjecting the above compound of formula (I-A) in which $R^{2-A}$ is t-butoxycarbonyl, that is the compound of formula (I-A-c):

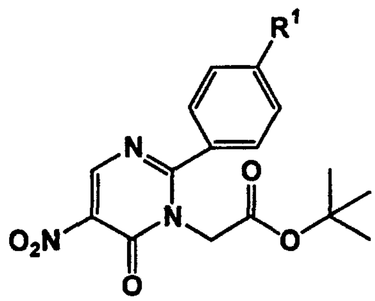

(I-A-c)

wherein all the symbols are as hereinbefore defined;
to deprotection under acidic conditions.

The reaction of deprotection under acidic conditions is carried out as hereinbefore defined.

(d) The compound of formula (I-C) may be prepared by subjecting the above compound of formula (I-A) in which $R^{2-A}$ is furan-2-yl, that is the compound of formula (I-A-d):

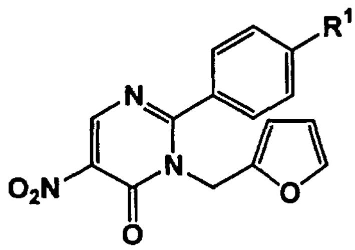

(I-A-d)

wherein all the symbols are as hereinbefore defined;
to oxidative cleavage.

The reaction of oxidative cleavage is carried out as hereinbefore defined.

(e) The compound of formula (I-C) may be prepared by subjecting the above compound of formula (I-A) in which $R^{2-A}$ is $-CH=CH_2$, that is the compound of formula (I-A-b):

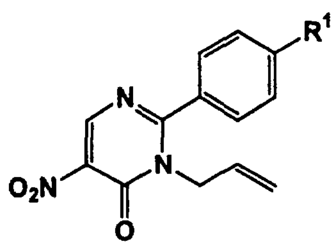

(I-A-b)

wherein all the symbols are as hereinbefore defined;
to oxidative cleavage using an oxidant.

The reaction of oxidative cleavage using an oxidant is known, for example, it may be carried out in an organic solvent (e.g. methylene chloride, chloroform, carbon tetrachloride, acetonitrile), water or a mixture thereof, in the presence of an oxidant (e.g. Ruthenium chloride, Ruthenium (IV) oxide, hydrogen peroxide solution), using a reagent for oxidative cleavage (e.g. ozone, potassium permanganate), at -78-50°C.
As will be apparent to those skilled in the art, reagents for oxidative cleavage or oxidants other than the above reagents are also preferred. For example, reagents described in Comprehensive Organic Transformations (Richard C. Larock, VCH Publishers, Inc., (1989) page 828 - 829), may be used.

(f) The compound of formula (I-C) may be prepared by subjecting the above compound of formula (I-B):

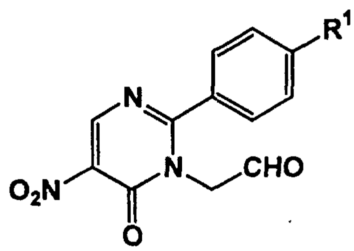 (I-B)

wherein all the symbols are as hereinbefore defined;
to oxidation.
The oxidation reaction of aldehyde to carboxylic acid is known, for example, it may be carried out in an organic solvent (e.g. t-butanol), water or a buffer (e.g. buffer of sodium dihydrogenphosphate, buffer of potassium dihydrogenphosphate), or a mixture thereof, in the presence or absence of olefin compound (e.g. 2-methyl-2-butene, 1-methylpropene), using an oxidant (e.g. sodium hypochlorite solution, potassium permanganate), at 0-50 °C.
As will be apparent to those skilled in the art, oxidants other than the above oxidants are also preferred. For example, reagents described in Comprehensive Organic Transformations (Richard C. Larock, VCH Publishers, Inc., (1989) page 838-840), may be used.

(g) The compound of formula (I-C) may be prepared by reacting a compound of formula (II-B):

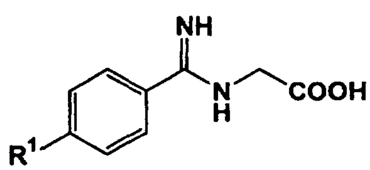 (II-B)

wherein $R^1$ is as hereinbefore defined;
with 3-methoxy-2-nitroacrylate compound of formula (III):

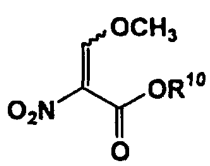 (III)

wherein $R^{10}$ is as hereinbefore defined.

The above reaction of the compound of the formula (II-B) and the compound of the formula (III), is carried out by the same procedure as the above reaction of amidine compound of formula (II-A) and 3-methoxy-2-nitroacrylate compound of formula (III).

[4] The compound in which $R^2$ is

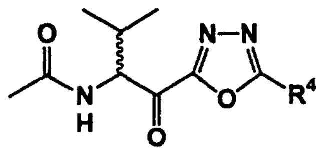

in the compounds of the present invention of formula (I), that is the compound of formula (I-D):

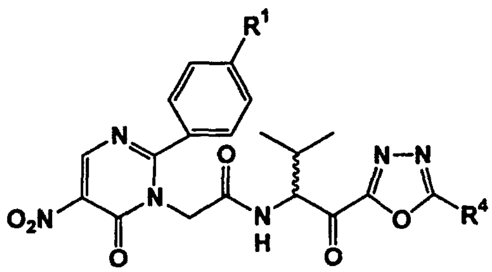

(I-D)

wherein all the symbols are as hereinbefore defined;
may be prepared by subjecting an above compound of formula (I-C) with the compound of formula (IV):

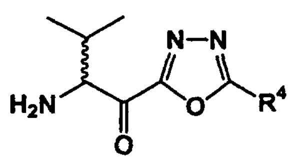

(IV)

wherein all the symbols are as hereinbefore defined;
to amidation.

The method of amidation is known. It includes the method

(1) via an acyl halide,
(2) via a mixed acid anhydride,
(3) using a condensing agent.

These methods are explained as follows.

(1) The method via an acyl halide may be carried out, for example, by reacting a compound of the formula (I-C) with an acyl halide (e.g. oxalyl chloride or thionyl chloride etc.) in an inert organic solvent (e.g. chloroform, methylene chloride, diethyl ether or tetrahydrofuran) or without a solvent at -20°C to reflux temperature. And then the obtained acyl halide derivative may be reacted with a compound of formula (IV) in an organic solvent (e.g. chloroform, methylene chloride, diethyl ether or tetrahydrofuran), in the presence of a tertiary amine (e.g. pyridine, triethyl amine, dimethyl aniline or dimethylaminopyridine) at -20-40°C.
(2) The method via a mixed acid anhydride may be carried out, for example, by reacting a compound of formula

(I-C) with an acyl halide (e.g. pivaloyl chloride, tosyl chloride, mesyl chloride), or an acid derivative (ethyl chloroformate or isobutyl chloroformate) in an inert organic solvent (e.g. chloroform, methylene chloride, diethyl ether, tetrahydrofuran, dimethylformamide) or without a solvent, in the presence of a tertiary amine (e.g. pyridine, triethylamine, dimethylaniline, dimethylaminopyridine, or N-methylmorpholine), at -20°C-40°C. And then the obtained mixed acid anhydride derivative may be reacted with a compound of formula (IV) in an inert organic solvent (e.g. chloroform, methylene chloride, diethyl ether or tetrahydrofuran), at -20-40°C.
(3) The method using a condensing agent may be carried out, for example, by reacting a compound of formula (I-C) with a compound of formula (V) in an organic solvent (e.g. chloroform, methylene chloride, dimethylformamide, diethyl ether or tetrahydrofuran) or without a solvent, in the presence or absence of a tertiary amine (e.g. pyridine, triethylamine, dimethylaniline or dimethylaminopyridine), using a condensing agent (e.g. 1, 3-dicyclohexyl carbodiimide (DCC), 1-ethyl-3-[3-(dimethylamino)propyl] carbodiimide (EDC), 1-ethyl-3-[3-(dimethylamino)propyl]carbodiimide (CDI), 2-chloro-1-methylpyridinium iodide), in the presence or absence of 1-hydroxybenzotiazole (HOBt), at 0-40°C.

The reaction described in (1), (2) and (3) may be carried out under an inert gas (e.g. argon, nitrogen) to avoid water in order to obtain a preferable result.
A preferable amidation of a compound of formula (IV) and a compound of formula (I-C) may be carried out by a method via a mixed acid anhydride.

[5] The known compound of formula (E):

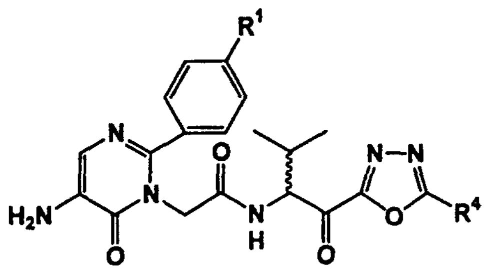
(E)

wherein all the symbols are as hereinbefore defined;
may be prepared by subjecting the compound of formula (I-D) to reduction.
The reduction of nitro group is known, for example, it may be carried out by a hydrogenolysis, a reduction using an organic metal.
The reaction of hydrogenation is known, for example, it may be carried out in an inert solvent [e.g. ether, e.g. tetrahydrofuran, dioxane, dimethoxyethane, diethyl ether; alcohol, e.g. methanol, ethanol; benzene, e.g. benzene, toluene; ketone, e.g. acetone, methyl ethyl ketone; nitrile, e.g. acetonitrile; amide, e.g. dimethylformamide; water, ethyl acetate, acetic acid or two more mixture thereof], in the presence of a catalyst (e.g. palladium on carbon, palladium black, palladium, palladium hydroxide, platinum dioxide, Nickel, Raney-nickel or Ruthenium chloride), in the presence or absence of an inorganic acid (e.g. hydrochloric acid, sulfuric acid, hypochlorous acid, boric acid or tetrafluoroboric acid) or an organic acid (e.g. acetic acid, p-toluenesulfonic acid, oxalic acid, trifluoroacetic acid or formic acid), at ordinary or elevated pressure of hydrogen gas or ammonium formate at 0-200°C.
In case of using an acid, the salt thereof may be used.
The reaction of reduction using an organic metal is known, for example, it may be carried out in a water soluble solvent (e.g. ethanol, methanol), in the presence or absence of hydrochloric acid, using an organic metal (e.g. zinc, iron, tin, tin chloride, iron chloride), at 50-150°C.
A preferable reaction of reduction of the compound of formula (I-D) is hydrogenation.
The compound of formula (II-A) and (II-B) may be prepared by a method described in EP 528633 (JP-A-5-286946), or a method in accordance with them.
The compound of formula (III) may be prepared by a method in accordance with a description in J. Heterocyclic Chem., 22, 337 (1985).
Racemate of the compound of formula (IV) was described in JP-A-11-066065. Racemate of the compound of formula (I-D) was able to be prepared easily, using them. Besides, an optically active compound of formula (IV) was

able to be prepared easily by a same procedure as the one described in JP-A-11-066065, using an available optically active valine derivative. An optically active compound of formula (I-D) was able to be prepared easily, using an optically active compound of formula (IV).

In each reaction in the present specification, products may be isolated, washed, dried, and purified, only suitable operation thereof or without these operations ahead of a next step. The product may be purified by conventional techniques, for example, purification may be carried out by distillation at an atmospheric or a reduced pressure, by high performance liquid chromatography, by thin layer chromatography or by silica gel or magnesium silicate column chromatography, by washing or by recrystallization.

The compounds of the present invention may be converted into the corresponding salts. Non-toxic salts and water-soluble salts are preferred. Suitable salts, for example, include: salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine).

The compounds of the present invention may be converted into the corresponding acid addition salts. Non-toxic acid addition salts and water-soluble acid addition salts are preferred. Suitable salts, for example, include: salts of inorganic acids e.g. hydrochloride, hydrobromide, sulfate, phosphate, nitrate; salts of organic acids e.g. acetate, trifluoroacetate, lactate, tartarate, oxalate, fumarate, maleate, citrate, benzoate, methanesulphonate, ethanesulphonate, benzenesulphonate, toluenesulphonate, isethionate, glucuronate, gluconate.

The compounds of the present invention or salts thereof may be converted into the corresponding hydrates by conventional means.

Effect

It has been confirmed that the compound of formula (I-D) of the present invention has an inhibitory activity on elastase. For example, the following results were obtained in laboratory tests.

(1) Inhibitory effects on human sputum elastase

A mixture of 0.5 ml of 0.2 mM HEPES buffer (pH 8.0), 0.2 ml of 2.5 M aqueous solution of sodium chloride, 0.1 ml of 1% polyethyleneglycol 6000, 0.13 ml of distilled water, 0.01 ml of a solution of test compound in dimethylsulfoxide (DMSO) and 0.05 ml of 0.8 U/ml human sputum elastase (HSE) was pre-incubated at 37°C, for 20 minutes. 0.01 ml of 5 mM MeO-Suc-Ala-Ala-Pro-Val-pNA in DMSO was added to the above mixture and the mixture was incubated at 37°C for 5 minutes. The reaction was terminated by 0.1 ml of 50% acetic acid and then p-nitroanilide (pNA) released was measured spectrophotometrically at 405 nm. Percent inhibition of a compound was calculated by the following equation.

$$\text{Inhibition (\%)} = [1 - \{\text{delta OD (test-blank) / delta OD (control-blank)}\}] \times 100$$

As a result, $IC_{50}$ of the compound of Example 7(1) was 88 nM.

(2) Inhibitory effects on human sputum elastase induced lung hemorrhage in hamster

A suspension of test compound in 0.5% carboxymethylcellulose sodium salt, 80% polyethyleneglycol 400 or 2% Tween 80 was administered orally to a group of 5 Syrian hamsters. At 60 minutes after the administration, 10 U/ 100 µl/ lung of HSE was injected intrabronchially via surgically exposed trachea under pentobarbital anesthesia (60 mg/kg, i.p.) to induce lung injury. At 60 minutes after the injection, hamsters were bled to sacrifice and bronchoalveolar was lavaged with 2.5 ml of saline and bronchoalveolar lavage fluid (BALF) was obtained. The obtained BALF (0.5 ml) was diluted by 4 times with 2% aqueous solution of sodium carbonate and sonicated for 10 seconds. Besides, the BALF was diluted by 2.5 times with 2% aqueous solution of sodium carbonate. The amount of blood in BALF was calculated from absorbance at 414 nm using standard curve.

Toxicity

The toxicity of the compounds of formula (I-D) of the present invention is very low and therefore the compounds may be considered safe for pharmaceutical use.

Application for Pharmaceuticals

The compound of formula (I-D) of the present invention or a non-toxic salt thereof has an inhibitory activity on elastase, and it is useful for treatment and / or prevention of a disease induced by an abnormal enhancement of degradation of elastin, collagen fiber and / or proteoglycans by elastase, for example, pulmonary emphysema, chronic obstructive pulmonary disease, rheumatoid arthritis, arteriosclerosis, adult respiratory distress syndrome (ARDS), glomerular nephritis, myocardial infarction, ulcerative colitis and gingivitis.

The compound of formula (I-D) of the present invention or a non-toxic salt thereof may be normally administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention may be administered in the form of, for example, solid forms for oral administration, liquid forms for oral administration, injections, liniments or suppositories for parenteral administration.

Solid forms for oral administration include compressed tablets, pills, capsules, dispersible powders, and granules. Capsules include hard capsules and soft capsules.

In such solid forms, one or more of the active compound(s) may be admixed with vehicles (such as lactose, mannitol, glucose, microcrystalline cellulose, starch), binders (such as hydroxypropyl cellulose, polyvinylpyrrolidone or magnesium metasilicate aluminate), disintegrants (such as cellulose calcium glycolate), lubricants (such as magnesium stearate), stabilizing agents, and solution adjuvants (such as glutamic acid or aspartic acid) and prepared according to methods well known in normal pharmaceutical practice. The solid forms may, if desired, be coated with coating agents (such as sugar, gelatin, hydroxypropyl cellulose or hydroxypropylmethyl cellulose phthalate), or be coated with two or more films. And further, coating may include absorbable materials contained in capsules, such as gelatin.

Liquid forms for oral administration include pharmaceutically acceptable solutions, suspensions and emulsions, syrups and elixirs. In such forms, one or more of the active compound(s) may be dissolved, suspended or emulsified into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

Injections for parenteral administration include sterile aqueous, suspensions, emulsions and solid forms that are dissolved or suspended into solvent(s) for injection immediately before use. In injections, one or more of the active compound(s) may be dissolved, suspended or emulsified into solvent(s). The solvents may include distilled water for injection, physiological salt solution, vegetable oil, propylene glycol, polyethyleneglycol, alcohol, e.g. ethanol, or a mixture thereof.

Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative. They may be sterilized at a final step, or may be prepared and compensated according to sterile methods. They may also be manufactured in the form of sterile solid forms, for example, freeze-dried products, which may be dissolved in sterile water or some other sterile diluent(s) for injection immediately before use.

Other forms for parenteral administration include liquids for external use, ointments and endermic liniments, inhalations, sprays, suppositories and pessaries for vaginal administration which comprise one or more of the active compound(s) and may be prepared by methods known per se. Sprays may comprise additional substances other than diluents, such as stabilizing agents (such as sodium sulfate), isotonic buffers (such as sodium chloride, sodium citrate or citric acid). For preparation of such sprays, for example, the method described in the United States Patent No. 2,868,691 or 3,095,355 may be used.

Best mode for carrying out the invention

The following reference examples and examples illustrate, but do not limit the present invention.

The solvents in parenthesis show the developing or eluting solvents and the ratios of the solvents used are by volume in chromatographic separations and TLC.

The NMR data are shown with the solvents used in the measurements in parentheses.

Examplel 1

Preparation of 1-(2,2-dimethoxyethyl)-6-oxo-2-phenyl-5-nitro-1,6-dihydropyrimidine

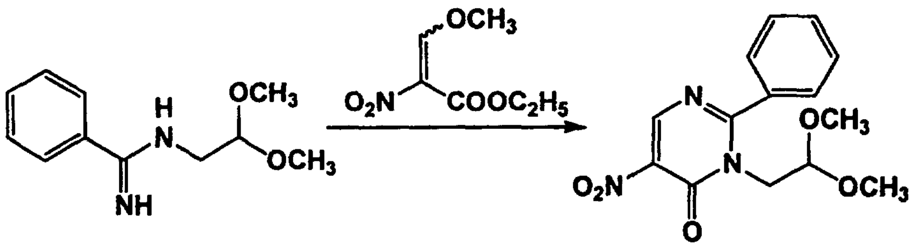

To a solution of N-(2,2-dimethoxyethy)phenylamidine (1.42 g) in methanol (40 ml), under an argon atmosphere, 3-methoxy-2-nitroacrylic acid ethyl ester (1 g) in methanol (23 ml) was dropped slowly, at room temperature. The mixture was stirred for 17 hours at room temperature. The reaction mixture was concentrated. The residue was purified by column chromatography on silica gel (hexane : ethyl acetate = 2 : 1 → 1 : 1) to give the title compound (0.824 g) having the following physical data.
TLC: Rf 0.57 (Hexane : Acetyl acetate = 1: 1);
NMR ($CDCl_3$): δ 8.97 (1H, s), 7.60-7.50 (5H, m), 4.86 (1H, t, J = 5.4Hz), 4.24 (2H, d, J = 5.4Hz), 3.33 (6H, s).

Example 2

Preparation of 1-(2-propenyl)-6-oxo-2-phenyl-5-nitro-1,6-dihydropyrimidine

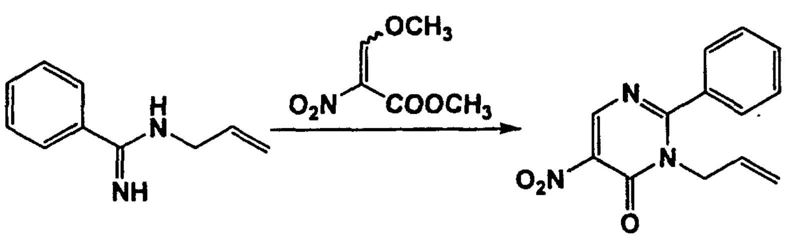

To a solution of N-(2-propenyl)phenylamidine (2.18 g) in toluene (62 ml), under an argon atmosphere, 3-methoxy-2-nitroacrylic acid methyl ester (2.0 g) in toluene (62 ml) was dropped over 2 hours at 30°C. The mixture was stirred for 2 hours at 30°C. To the reaction mixture, methanol (15 ml) was added. The solution was washed successively with 1N hydrochloric acid (70 ml), a saturated aqueous solution of sodium bicarbonate (30 ml), water (30 ml) and a saturated aqueous solution of sodium chloride (30 ml), and concentrated. The obtained solid was dissolved in ethyl acetate (8.1 ml) with heating, and was subjected to recrystallization. Precipitated crystals were obtained by filtration, washed with water and dried to give the title compound (2.48 g) having the following physical data.
TLC: Rf 0.30 (Hexane : Acetyl acetate = 7 : 3);
NMR ($CDCl_3$): δ 8.96 (1H, s), 7.65-7.50 (5H, m), 5.90 (1H, m), 5.30 (1H, dd, J = 10.2, 0.8Hz), 5.04 (1H, dd, J = 16.6, 0.8Hz), 4.67 (2H, dt, J = 5.4, 0.8 Hz).

Example 3(1)

Preparation of 1-(t-butoxycarbonylmethyl)-6-oxo-2-phenyl-5-nitro-1,6-dihydropyrimidine

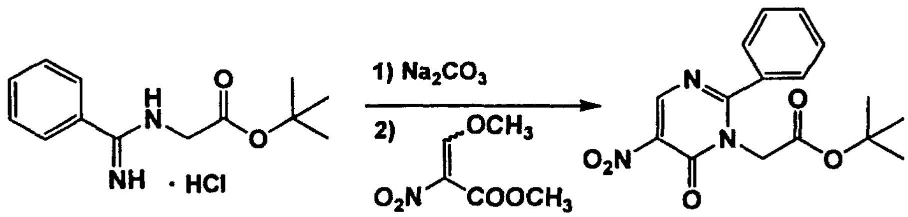


To a solution of sodium carbonate (25 g) in water (145 ml) and toluene (70 ml), N-(t-butoxycarbonylmethyl) phenylamidine hydrochloride (6.5 g) was added. The mixture was stirred for 5 minutes at room temperature. The reaction mixture was extracted twice with toluene (70 ml). The extract was dried over anhydrous sodium sulfate and filtered to give the solution of free compound of N-(t-butoxycarbonylmethyl)phenylamidine in toluene. To this solution, under atmosphere of argon, a solution of 3-methoxy-2-nitroacrylic acid methyl ester (3.17 g) in toluene (40 ml) was dropped over 1 hour. The mixture was stirred for 1 hour at 30°C, and then for 1.5 hours at 50°C. To the reaction mixture, methanol (15 ml) was added. The solution was successivelywashed with 1N hydrochloric acid, water, a saturated aqueous solution of sodium bicarbonate and a saturated aqueous solution of sodium chloride, dried over anhydrous sodium sulfate and concentrated. The residue was recrystallized from isopropanol to give the title compound (4.2 g) having the following physical data.
TLC: Rf 0.23 (Hexane : Acetyl acetate = 7 : 3);
NMR ($CDCl_3$): δ 9.00 (1H, s), 7.70-7.50 (5H, m), 4.63 (2H, s), 1.46 (9H, s).

Example 3(2)

Preparation of 1-(t-butoxycarbonylmethyl)-6-oxo-2-phenyl-5-nitro-1,6-dihydropyrimidine

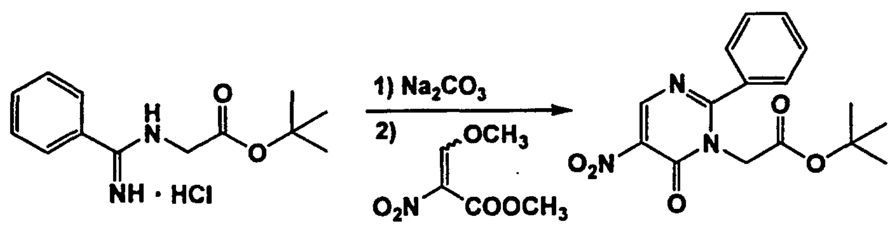


To a solution of deaerated sodium carbonate (272 g) in water (1500 ml), N-(t-butoxycarbonylmethyl)phenylamidine hydrochloride (72 g) was added. The mixture was extracted three times with toluene (300 ml). The extract was dried over anhydrous sodium sulfate and filtered to give a solution of a free compound of N-(t-butoxycarbonylmethyl)phenylamidine in toluene. This solution was warmed to 30°C, and a solution of 3-methoxy-2-nitroacrylic acid methyl ester (35.6 g) in toluene (300 ml) was dropped into the solution over 50 minutes. An internal temperature of the solution was warmed to 45-50°C, and then the solution was stirred for 1 hour. The completion of the reaction was confirmed by HPLC. The reaction mixture was cooled to room temperature, and insoluble oil separated was dissolved by addition of methanol (about 50 ml). The solution was washed successively with 1N hydrochloric acid (600 ml x 2 times), water, a saturated aqueous solution of sodium bicarbonate, water and a saturated aqueous solution of sodium chloride, dried over anhydrous sodium sulfate, and was concentrated to give crude crystals of 1-(t-butoxycarbonylmethyl)-6-oxo-2-phenyl-5-nitro-1,6-dihydropyrimidine (69 g).

The crude crystals were dissolved in isopropanol (500 ml) with heating, and the solution was stirred for 2 hours at room temperature, and then for 5 hours at 5°C. The crystals were obtained by filtration, washed with cooled isopropanol (300 ml), and was dried under reduced pressure at room temperature to give the title compound (45.4 g) having the same physical data as those of the compound prepared in Example 3(1).

Example 4

Preparation of 1-(furan-2-ylmethyl)-6-oxo-2-phenyl-5-nitro-1,6-dihydropyrimidine

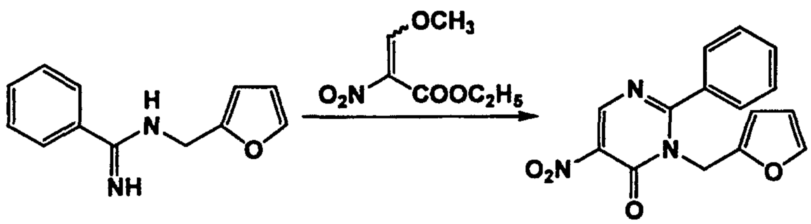

To a solution of N-(furan-2-ylmethyl)phenylamidine (10.32 g) in toluene (300 ml), under atmosphere of argon, a solution of 3-methoxy-2-nitroacrylic acid ethyl ester (9.03 g) in toluene (250 ml) was dropped slowly, at 100°C. The mixture was stirred for 2 hours at 100°C. The reaction mixture was cooled to room temperature, washed with 1N hydrochloric acid (150 ml), a saturated aqueous solution of sodium bicarbonate (150 ml), water (150 ml) and a saturated aqueous solution of sodium chloride (150 ml), successively, dried over anhydrous sodium sulfate and concentrated. The residual solid was recrystallized from ethyl acetate (100 ml). Precipitated crystals were obtained by filtration, washed with ethyl acetate and dried to give the title compound (7.6 g) having the following physical data.
TLC: Rf 0.42 (Hexane : Acetyl acetate = 3 : 2);
NMR ($CDCl_3$): δ 8.94 (1H, s), 7.66-7.53 (5H, m), 7.32 (1H, t, J = 1.5Hz), 6.31 (2H, d, J = 1.5Hz), 5.28 (2H, s).

Example 5(1)

Preparation of 1-(formylmethyl)-6-oxo-2-phenyl-5-nitro-1,6-dihydropyrimidine

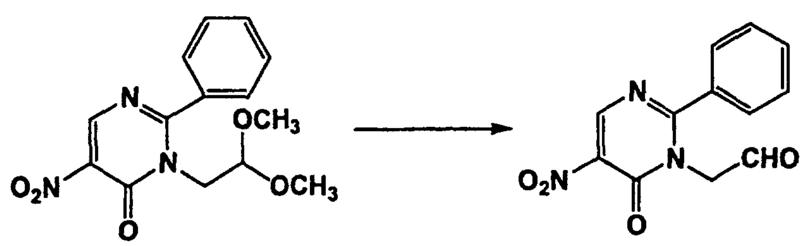

A mixed solution of the compound prepared in Example 1 (100 mg) in water (0.1 ml) and trifluoroacetic acid (0.4 ml), under an argon atmosphere, was stirred for 30 minutes at room temperature. The reaction mixture was concentrated to give the title compound (84 mg) having the following physical data.
TLC: Rf 0.26 (Hexane : Acetyl acetate = 1: 1);
NMR ($CDCl_3$): δ 9.65 (1H, s), 9.10 (1H, s), 7.75-7.40 (5H, m), 4.96 (2H, s).

Example 5(2)

Preparation of 1-(formylmethyl)-6-oxo-2-phenyl-5-nitro-1,6-dihydropyrimidine

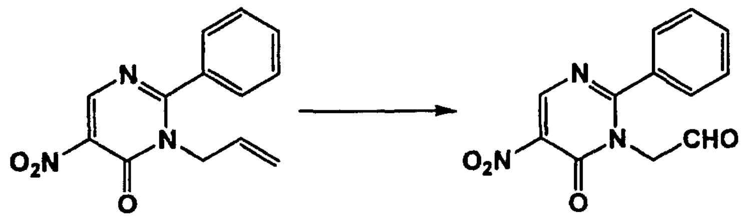

To a solution of the compound prepared in Example 2 (514 mg) in methylene chloride (18 ml) and methanol (2 ml), ozone was introduced at -70°C until a color of the solution changed blue. The reaction mixture was bubbled with argon at -70°C, and then was added dimethylsulfide (0.179 ml). The reaction mixture was warmed to room temperature, and was added water (1 ml) and t-butanol (10 ml). The mixture was concentrated. The residue was recrystallized from acetonitrile to give the title compound (400 mg) having the same physical data as those of the compound prepared in Example 5(1).

Example 5(3)

Preparation of 1-(formylmethyl)-6-oxo-2-phenyl-5-nitro-1,6-dihydropyrimidine

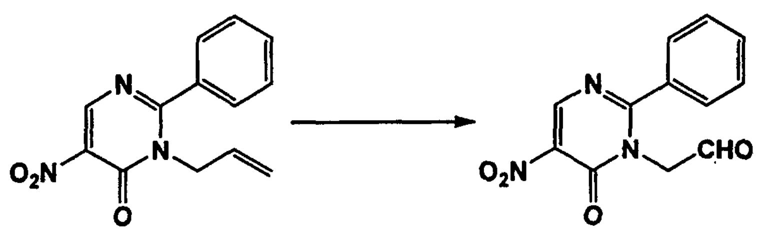

To a solution of the compound prepared in Example 2 (11.78 g) in methylene chloride (900 ml), was added t-butanol (23 ml). To the solution, ozone was introduced at -70°C until a color of the solution changed blue. Methanol (2 ml) was dropped into the solution to give white precipitation. The remaining ozone was removed by sequential bubbling of oxygen and argon for 30 minutes each at -70°C. Dimethylsulfide (4.1 ml) was added to the solution. The solution was warmed to room temperature, and were added t-butanol (50 ml) and water (1 ml). The solution was concentrated under reduced pressure at about 22°C to give crude compound of 1-(formylmethyl)-6-oxo-2-phenyl-5-nitro-1,6-dihydropyrimidine (12.23 g).

The crude crystals were recrystallized from acetonitrile (45 ml) to give the title compound (7.7 g) having the same physical data as those of the compound prepared in Example 5(1). Besides, concentrated mother liquor was recrystallized from a mixed solvent of acetonitrile (10 ml) and ethyl acetate (25 ml) to give the title compound (1.0 g).

Example 6(1)

Preparation of 2-(6-oxo-2-phenyl-5-nitro-1,6-dihydropyrimidin-1-yl)acetic acid

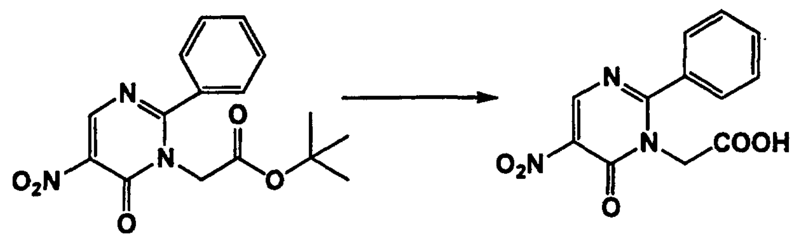

A solution of the compound prepared in Example 3(1) or Example 3(2) (5 g) in trifluoroacetic acid (10 ml) was stirred for 4 hours at room temperature. The reaction mixture was concentrated, and washed with ethyl acetate to give the title compound (3.8 g) having the following physical data.
TLC: Rf 0.16 (Chloroform : Methanol: Acetic acid = 90 : 10 : 1);
NMR (DMSO-$d_6$): δ 9.07 (1H, s), 7.70-7.55 (5H, m), 4.59 (2H, s).

Example 6(2)

Preparation of 2-(6-oxo-2-phenyl-5-nitro-1,6-dihydropyrimidin-1-yl)acetic acid

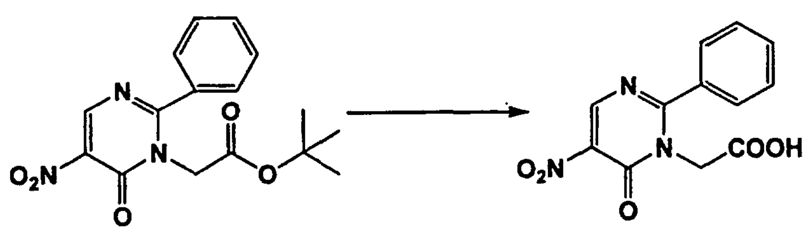

A solution of the compound prepared in Example 3(1) or Example 3(2) (43 g) in 4N hydrochloride - acetic acid (129 ml) was stirred for 5 hours at room temperature (20°C), under an argon atmosphere. The completion of the reaction was confirmed by HPLC. t-Butyl methyl ether (200 ml) was added to the reaction mixture, and the mixture was stirred for 30 minutes in a water-bath. The precipitates were collected by filtration, washed twice with t-butyl methyl ether (100 ml), dried under reduced pressure at room temperature to give crude crystals of 6-oxo-2-phenyl-5-nitro-1,6-dihydropyrimidine-1-ylmethylcarbonic acid (41 g).

A suspension of the crude crystals in t-butyl methyl ether (600 ml) was refluxed for 1 hour, and then about 100 mL of t-butyl methyl ether was distilled off. To the reaction mixture was added t-Butyl methyl ether (150 mL), and the mixture was refluxed for 6 hours. The reaction mixture was cooled for 30 minutes in an ice-bath. The precipitates were collected by filtration, washed with t-butyl methyl ether and dried for 16 hours under reduced pressure at room temperature to give the title compound (35.4 g) having the same physical data as those of the compound prepared in Example 6(1).

Example 6(3)

Preparation of 2-(6-oxo-2-phenyl-5-nitro-1,6-dihydropyrimidin-1-yl)acetic acid

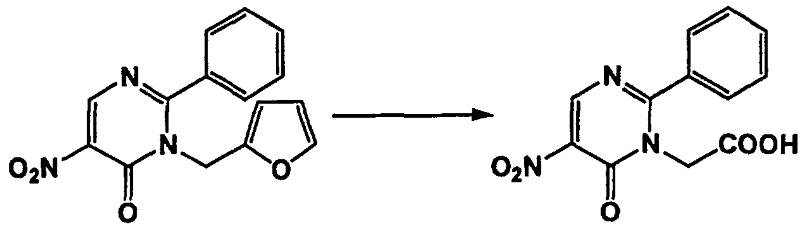

To a solution of the compound prepared in Example 4 (297 mg) in methylene chloride (18 ml) and methanol (7 ml), ozone was blown through at -70°C until a color of the solution was changed to blue. The reaction mixture was blown through argon at -70°C, and then dimethylsulfide (1.0 ml) was added to. The reaction mixture was warmed to room temperature, and concentrated. The residue was purified by column chromatography on silica gel (methylene chloride : methanol = 10 : 1 → methylene chloride : methanol: acetic acid = 20 : 2 : 1) to give the title compound (48 mg) having the same physical data as those of the compound prepared in Example 6(1).

Example 6(4)

Preparation of 2-(6-oxo-2-phenyl-5-nitro-1,6-dihydropyrimidin-1-yl)acetic acid

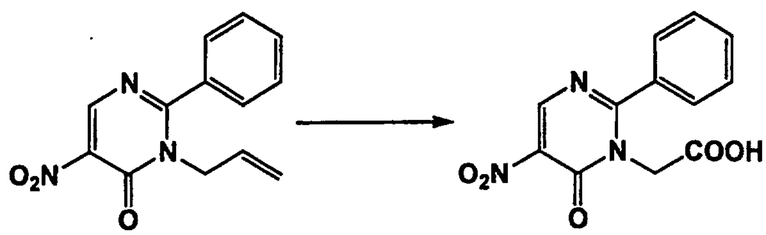

To a solution of the compound prepared in Example 2 (257 mg) in carbon tetrachloride (3 ml) and acetonitrile (3 ml), water (3 ml), were added sodium periodate (1.07 g) and ruthenium trichloride (0.2 mg). The mixture was stirred for 1.5 hours at room temperature. The reaction mixture was filtered, and the filtrate was diluted with ethyl acetate. The solution was washed with water and a saturated aqueous solution of sodium chloride, and was dried over anhydrous magnesium sulfate and concentrated. A mixed solvent of hexane and ethyl acetate (2 : 3) was added to the residue. The precipitates were collected by filtration and dried to give the title compound (107 mg) having the same physical data as those of the compound prepared in Example 6(1).

Example 6(5)

Preparation of 2-(6-oxo-2-phenyl-5-nitro-1,6-dihydropyrimidin-1-yl)acetic acid

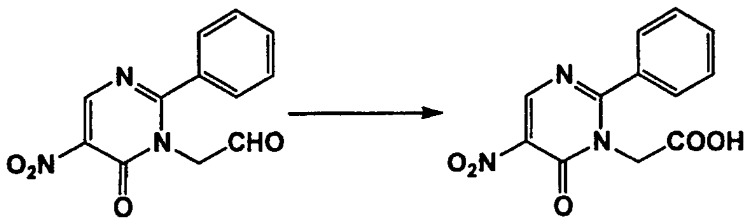

To a solution of the compound prepared in Example 5(1), 5(2) or 5(3) (5.91 g) in t-butanol (22.8 ml) and water (5.7 ml), a sodium dihydrogenphosphate buffer [an aqueous solution of sodium dihydrogenphosphate dihydrate (3.28 g/water 9.12 ml)] and 2-methyl-2-butene (7.2 g) were added. The mixture was cooled to 5°C, and then an aqueous solution of sodium chlorite [sodium chlorite (6.19 g, 80 % containing) in water (11.4 ml)] was added to. The mixture was stirred for 4 hours at room temperature. To the reaction mixture, 4N aqueous solution of sodium hydroxide (5.7 ml) was added, and the mixture was concentrated. 2N hydrochloric acid (17.1 ml) and t-butanol (9.6 ml) was added to the residue. Precipitated crystals were obtained by filtration. 2N hydrochloric acid (17.1 ml) was added to the crystals, and the mixture was stirred for 2.5 hours. Precipitated crystals obtained by filtration was dried to give the title compound (5.82 g) having the same physical data as those of the compound prepared in Example 6(1).

Example 6(6)

Preparation of 2-(6-oxo-2-phenyl-5-nitro-1,6-dihydropyrimidin-1-yl)acetic acid

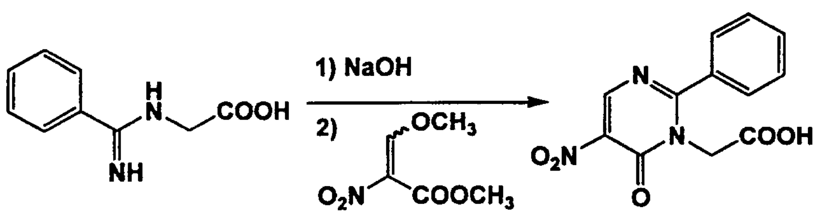


To a hot solution of sodium hydroxide (44 mg) in methanol (0.8 ml), were added N-(2-carboxymethyl)phenylamidine (196 mg) and toluene in this order. The toluene was distilled off. The residue was dissolved in isopropanol (0.5 ml) with heating, and filtered. Diisopropyl ether (0.8 ml) was added to the filtrate slowly. This solution was allowed to stand at about 30°C with stirring. Precipitated crystals were obtained by filtration, dried under reduced pressure at room temperature to give N-(2-carboxymethyl)phenylamidine sodium salt (220 mg).

Into a suspension of the above compound in dimethylformamide (10 ml), a solution of 3-methoxy-2-nitroacrylic acid methyl ester (161 mg) in toluene (1 ml) was dropped slowly at 30°C, and the mixture was stirred for 1 hour. The reaction mixture was poured into 2N hydrochloric acid (15 ml), and was extracted three times with ethyl acetate (30 ml). The extract was washed successively with 2N hydrochloric acid (15 ml x 2 times) and a saturated aqueous solution of sodium chloride (15 ml x 2 times), dried over anhydrous magnesium sulfate and concentrated. A solution of the residue in t-butyl methyl ether (10 ml) was stirred for 30 minutes at 50°C. Precipitated crystals were obtained by filtration at room temperature to give the title compound (151 mg) having the same physical data as those of the compound prepared in Example 6(1).

Example 7(1)

Preparation of N-[(1RS)-1-(5-(t-butyl)-1,3,4-oxadiazol-2-ylcarbonyl)-2-methylpropyl]-2-[6-oxo-2-phenyl-5-nitro-1,6-dihydropyrimidin-1-yl]acetamide

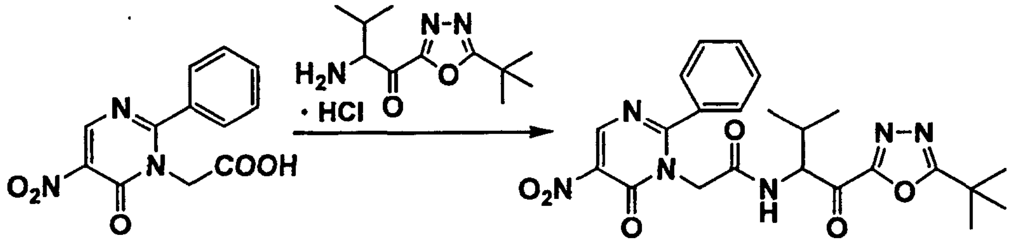


To a solution of the compound prepared in Example 6(1), 6(2), 6(3), 6(4), 6(5) or 6(6) (1.1 g) in tetrahydrofuran (20 ml), N-methylmorpholine (0.44 ml) was added at -20°C. To the mixture, ethyl chlorocarbonate (434 mg) was added at -20°C. The mixture was stirred for 30 minutes at that temperature. To the reaction mixture, (1RS)-1-(5-(t-butyl)-1,3,4-oxadiazol-2-ylcarbonyl)-2-methylpropylamine hydrochloride (1.05 g) and N-methylmorpholine (0.44 ml) were added. The mixture was stirred for 2 hours at 0°C. The reaction mixture was diluted with ethyl acetate. The dilute was washed successively with 1N hydrochloric acid, a saturated aqueous solution of sodium bicarbonate, water and a saturated aqueous solution of sodium chloride, dried over anhydrous magnesium sulfate and concentrated. The residue was recrystallized from ethanol to give the title compound (79 mg) having the following physical data.
TLC: Rf 0.20 (Hexane : Acetyl acetate = 11 : 9);
NMR ($CDCl_3$): δ 8.97 (1H, s), 7.75-7.45 (5H, m), 7.06 (1H, d, J = 8.6Hz), 5.28 (1H, dd, J = 8.6, 5.9Hz), 4.91 (1H, d, J = 15.7Hz), 4.75 (1H, d, J = 15.7Hz), 2.60-2.35 (1H, m), 1.45 (9H, s), 1.03 (3H, d, J = 6.7Hz), 0.89 (3H, d, J = 6.7Hz).

Example 7(2)

Preparation of N-[(1RS)-1-(5-(t-butyl)-1,3,4-oxadiazol-2-ylcarbonyl)-2-methylpropyl]-2-[6-oxo-2-phenyl-5-nitro-1,6-dihydropyrimidin-1-yl]acetamide

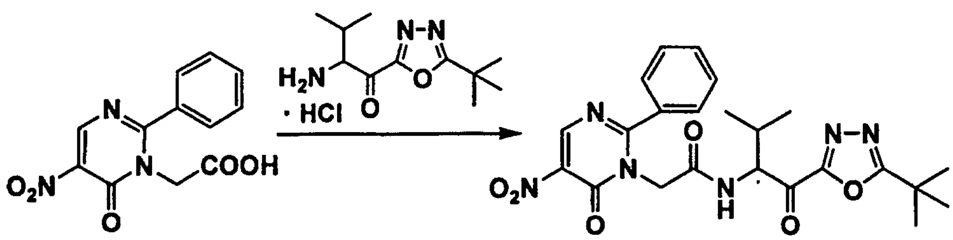


The compound prepared in Example 6(1), 6(2), 6(3), 6(4), 6(5) or 6(6) (28 g) was dissolved in dimethylformamide (203 ml) under an argon atmosphere, and then the solution was cooled to -20°C. N-methylmorpholine (12.6 ml) was added to the cooled solution. Ethyl chlorocarbonate (10.7 ml) was dropped into the mixture with keeping at -20 - -18°C, and then the mixture was stirred for 1 hour at that temperature. To the reaction mixture, (1RS)-1-(5-(t-butyl)-1,3,4-oxadiazol-2-ylcarbonyl)-2-methylpropylamine hydrochloride (31.4 g) was added, and then N-methylmolpholine (12.6 ml) was dropped into the mixture with keeping at -20 - -19°C. After the mixture was stirred for 1 hour at -20°C, dimethylformamide (50 ml) was added to the mixture, and then the mixture was warmed to 0°C over 2 hours. Ethyl acetate (1200 ml) was added to the reaction mixture. The solution was washed successively with 1N hydrochloric acid (300 ml), water (300 ml), a saturated aqueous solution of sodium bicarbonate, water (300 ml) and a saturated aqueous solution of sodium chloride, dried over anhydrous magnesium sulfate and concentrated to give crude crystals of N-[(1RS)-1-(5-(t-butyl)-1,3,4-oxadiazol-2-ylacrbonyl)-2-methylpropyl]-2- [6-oxo-2-phenyl-5-nitro-1,6-dihydropyrimidin-1-yl] acetamide (54 g).

A solution of crude crystals in ethanol (700 ml) was stirred for 2 hours at room temperature (18-20°C), and then for 2 hours at 5°C. A precipitated crystal obtained by filtration was washed with ethanol (about 200 ml) and dried for 12 hours under reduced pressure at room temperature to give the title compound (41.5 g) having the same physical data as those of the compound prepared in Example 7(1).

Example 8(1)

Preparation of N-[(1RS)-1-(5-(t-butyl)-1,3,4-oxadiazol-2-ylcarbonyl)-2-methylpropyl]-2-[6-oxo-2-phenyl-5-nitro-1,6-dihydropyrimidin-1-yl]acetamide

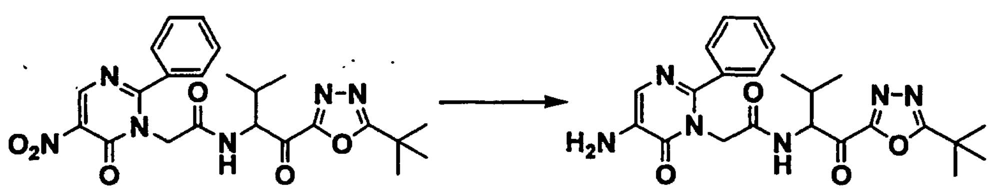

To a solution of the compound prepared in Example 7(1) or 7(2) (22 mg) in ethyl acetate (0.5 ml), 10% palladium-carbon (3 mg) was added. The mixture was stirred for 24 hours at room temperature, under an atmosphere of hydrogen gas. Methanol was added to the reaction mixture, and filtered through Celite ™. The filtrate was concentrated. The residue was purified by column chromatography on silica gel (methylene chloride : ethyl acetate : ethanol = 10 : 10 : 1) to give the title compound (11 mg) having the following physical data.
TLC: Rf 0.60 (Dichloromethane : Acetyl acetate : ethanol = 10: 10 : 1);
NMR ($CDCl_3$): δ 7.60-7.35 (6H, m), 6.92 (1H, d, J = 8.2Hz), 5.44 (1H, dd, J = 8.2, 4.9Hz), 4.68 (1H, d, J = 15.4Hz), 4.58 (1H, d, J = 15.4Hz), 2.64-2.40 (1H, m), 1.48 (9H, s), 1.07 (3H, d, J = 6.8Hz), 0.88 (3H, d, J = 6.8Hz).

Example 8(2)

Preparation of N-[(1RS)-1-(5-(t-butyl)-1,3,4-oxadiazol-2-ylcarbonyl)-2-methylpropyl]-2-[6-oxo-2-phenyl-5-nitro-1,6-dihydropyrimidin-1-yl]acetamide

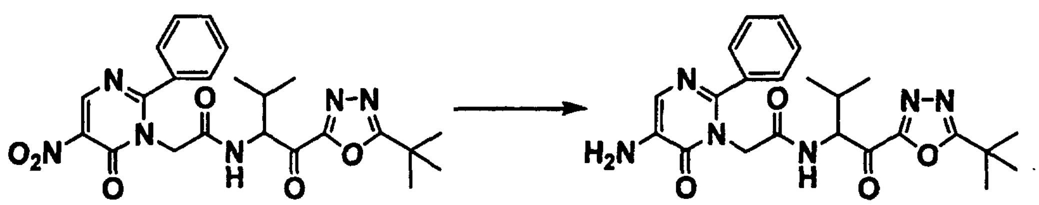

The compound prepared in Example 7(1) or 7(2) (4.5 g) was dissolved in tetrahydrofuran (27.9 ml) with heating at 50°C, and then water (3.1 ml) was added to. After the gaseous phase was replaced with argon at 50°C, 10% palladium-carbon (900 mg) was added to the mixture. The mixture was stirred for 2 hours at 400 rpm, under an atmosphere of hydrogen gas (5 atmospheric pressure). Palladium-carbon was removed by filtration, and was washed with tetrahydrofuran. The filtrate was concentrated. A solution of the residue in ethyl acetate (20 ml) was stirred for 20 minutes. Toluene (40 ml) was added to reaction solution. Additionally, toluene (20 ml) was added twice with an interval of 20 minutes, and the resulting mixture was stirred for 30 minutes. A precipitated crystal obtained by filtration was washed with toluene and dried for 12 hours under reduced pressure at room temperature to give crude crystals of N-[(1RS)-1-(5-(t-butyl)-1,3,4-oxadiazol-2-ylcarbonyl)-2-methylpropyl]-2-[6-oxo-2-phenyl-5-amino-1,6-dihydropyrimidin-1-yl] acetamide (2.92 g).

The crude crystals was dissolved in methanol (55 ml) at 40°C, and then the temperature was cooled to 30°C. To the mixture were added water (55 ml) and seed crystal, and the mixture was stirred for about 1 hour. The resulting

mixture was stirred for 3 hours in an ice-bath. Precipitated crystals were obtained by filtration, washed with water, and dried under reduced pressure for 3 hours at room temperature for 15 hours at 60°C to give the title compound (2.54 g) having the same physical data as those of the compound prepared in Example 8(1).

Formulation example

Formulation example 1

The following components were admixed in conventional method and punched out to obtain 100 tablets each containing 50 mg of active ingredient.

- N-[(1RS)-1-(5-(t-butyl)-1,3,4-oxadiazol-2-ylcarbonyl)-2-methylpropyl]-2-[6-oxo -2-phenyl-5-nitro-1,6-dihydropyrimidin-1-yl]acetamide (This is the compound prepared in Example 7(1)) 5.0 g
- Carboxymethylcellulose calcium (disintegrating agent) 0.2 g
- Magnesium stearate (lubricating agent) 0.1 g
- Microcrystaline cellulose 4.7 g

Formulation example 2

The following components were admixed in conventional method. The solution was sterilized in conventional manner, placed 5 ml portions into ampoules and freeze-dried to obtain 100 ampoules each containing 20 mg of the active ingredient.

- N-[(1RS)-1-(5-(t-butyl)-1,3,4-oxadiazol-2-ylcarbonyl)-2-methylpropyl]-2-[6-oxo -2-phenyl-5-nitro-1,6-dihydropyrimidin-1-yl]acetamide (This is the compound prepared in Example 7(1)) 2.0 g
- Mannitol 20 g
- Distilled water 500 ml

## Claims

**1.** A pyrimidine compound of formula (I)

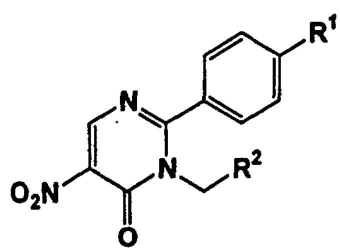

(I)

wherein $R^1$ is hydrogen or fluorine,
$R^2$ is $-CH(OR^3)_2$, t-butoxycarbonyl, $-CH=CH_2$, furan-2-yl, -CHO, -COOH or

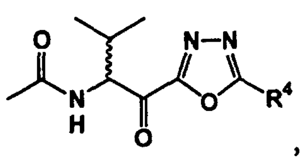

,

$R^3$ is methyl or ethyl,
$R^4$ is methyl, isopropyl, n-butyl, t-butyl, phenyl, 1-methylcyclopropyl, benzyl, 3-methylbenzyl, 3-trifluoromethylbenzyl, 3,4-methylenedioxybenzyl, α,α-dimethylbenzyl, a,a-dimetyl-3-methylbenzyl, α,α-dimetyl-3-trifluoromethylbenzyl or α,α-dimetyl-3,4-methylenedioxybenzyl;
or a non-toxic salt thereof.

**2.** A compound according to claim 1, wherein $R^2$ is $-CH(OR^3)_2$, t-butoxycarbonyl, $-CH=CH_2$ or furan-2-yl, that is the compound of formula (I-A)

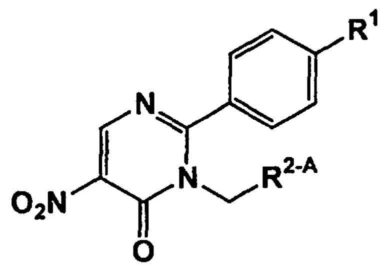

**(I-A)**

wherein $R^{2-A}$ is $-CH(OR^3)_2$, t-butoxycarbonyl, $-CH=CH_2$ or furan-2-yl, and $R^1$ is as defined in claim 1.

**3.** A compound according to claim 1, wherein $R^2$ is CHO, that is the compound of formula (I-B)

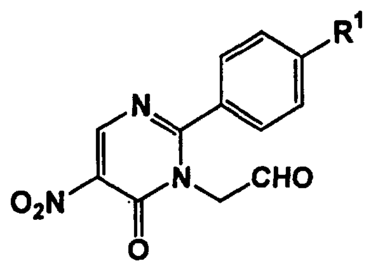

**(I-B)**

wherein $R^1$ is as defined in claim 1.

**4.** A compound according to claim 1, wherein $R^2$ is COOH, that is the compound of formula (I-C)

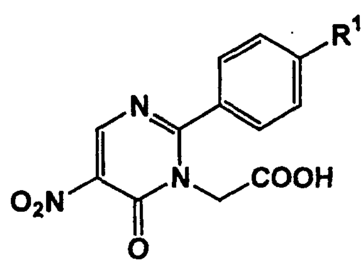

**(I-C)**

wherein $R^1$ is as defined in claim 1.

**5.** A compound according to claim 1, wherein $R^2$ is

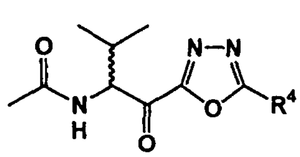

,

that is the compound of formula (I-D)

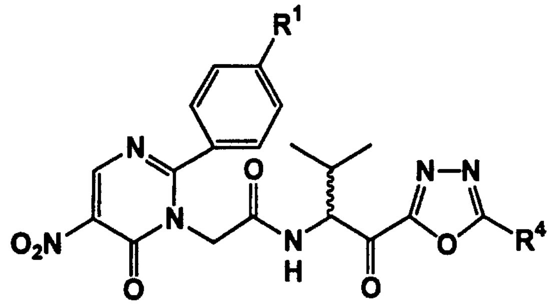 (I-D)

wherein all the symbols are as defined in claim 1.

**6.** The compound according to claim 2, which is selected from

(1) 1-(2,2-dimethoxyethyl)-6-oxo-2-phenyl-5-nitro-1,6-dihydropyrimidine,
(2)1-(t-butoxycarbonylmethyl)-6-oxo-2-phenyl-5-nitro-1,6-dihydropyrimidine,
(3) 1-(2-propenyl-6-oxo-2-phenyl-5-nitro-1,6-dihydropyrimidine and
(4) 1-(furan-2-ylmethyl)-6-oxo-2-phenyl-5-nitro-1,6-dihydropyrimidine.

**7.** The compound according to claim 3, that is 1-(formylmethyl)-6-oxo-2-phenyl-5-nitro-1,6-dihydropyrimidine.

**8.** The compound according to claim 4 is 2-(6-oxo-2-phenyl-5-nitro-1,6-dihydropyrimidin-1-yl)acetic acid.

**9.** The compound according to claim 5, that is N- [(1RS)-1-(5-(t-butyl)-1,3,4-oxadiazol-2-ylcarbonyl)-2-methylpropyl]-2-[6-oxo-2-phenyl-5-nitro-1,6-dihydropyrimidin-1-yl]acetamide.

**10.** A process for the preparation of a pyrimidine compound of formula (I-A) described in claim 2, which is **characterized by** reacting a compound of formula (II-A):

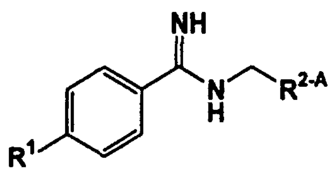 (II-A)

wherein all the symbols are as defined in claim 2;
with 3-methoxy-2-nitroacrylate compound of formula (III):

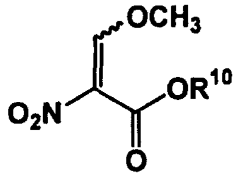 (III)

wherein $R^{10}$ is methyl or ethyl.

**11.** A process for the preparation of a pyrimidine compound of formula (I-B) described in claim 3, which is **characterized by** subjecting a compound wherein $R^{2-A}$ is $-CH(OR^3)_2$ in the compound of formula (I-A) described in claim 2, that is the compound of formula (I-A-a):

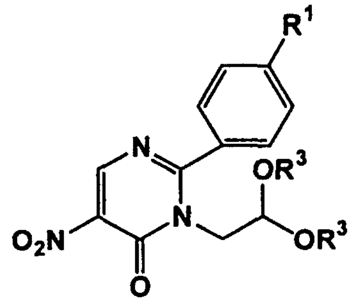

(I-A-a)

wherein all the symbols are as defined in claim 1;
to deprotection under an acidic condition.

**12.** A process for the preparation of a pyrimidine compound of formula (I-B) described in claim 3, which is **characterized by** subjecting a compound wherein $R^{2-A}$ is $-CH=CH_2$ in the compound of formula (I-A) described in claim 2, that is the compound of formula (I-A-b):

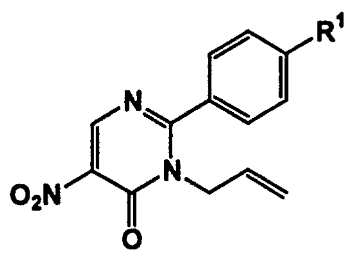

(I-A-b)

wherein all the symbols are as defined in claim 1;
to oxidative cleavage.

**13.** A process for the preparation of a pyrimidine compound of formula (I-C) described in claim 4, which is **characterized by** subjecting a compound wherein $R^{2-A}$ is t-butoxycarbonyl in the compound of formula (I-A) described in claim 2, that is the compound of formula (I-A-c):

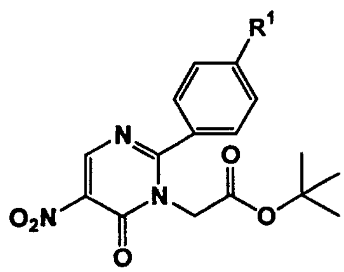

(I-A-c)

wherein all the symbols are as defined in claim 1;
to deprotection under an acid condition.

**14.** A process for the preparation of a pyrimidine compound of formula (I-C) described in claim 4, which is **characterized by** subjecting a compound wherein $R^{2-A}$ is furan-2-yl in the compound of formula (I-A) described in claim 2, that is the compound of formula (I-A-d):

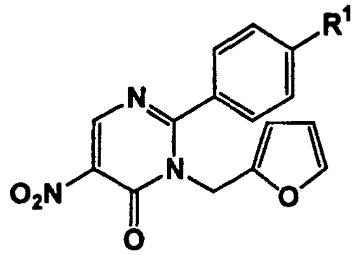

(I-A-d)

wherein all the symbols are as defined in claim 1;
to oxidative cleavage.

**15.** A process for the preparation of a pyrimidine compound of formula (I-C) described in claim 4, which is **characterized by** subjecting a compound wherein $R^{2-A}$ is $-CH=CH_2$ in the compound of formula (I-A) described in claim 2, that is the compound of formula (I-A-b):

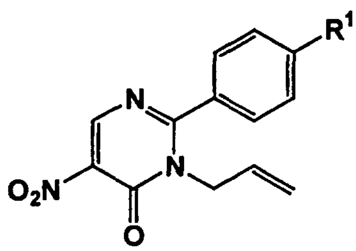

(I-A-b)

wherein all the symbols are as defined in claim 1;
to oxidative cleavage using an oxidant.

**16.** A process for the preparation of a pyrimidine compound of formula (I-C) described in claim 4, which is **characterized by** subjecting a compound of formula (I-B) described in claim 3 to oxidation.

**17.** A process for the preparation of a pyrimidine compound of formula (I-C) described in claim 4, which is **characterized by** reacting a compound of formula (II-B):

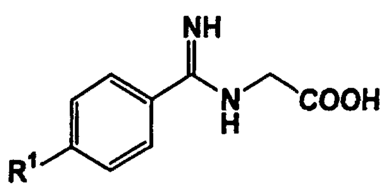

(II-B)

wherein all the symbols are as defined in claim 1;
with 3-methoxy-2-nitroacrylate compound of formula (III):

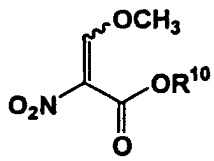

(III)

wherein $R^{10}$ is as hereinbefore defined.

**18.** A process for the preparation of a pyrimidine compound of formula (I-D) described in claim 5, which is **characterized by** subjecting a compound of formula (I-C) described in claim 4, with a compound of formula (IV):

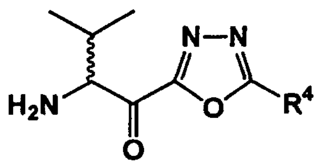

(IV)

wherein all the symbols are as defined in claim 1;
to amidation.

**19.** A process for the preparation of a pyrimidine compound of formula (E)

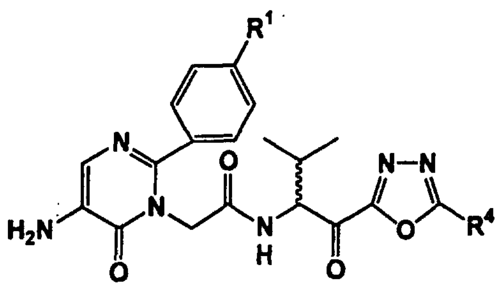

(E)

wherein all the symbols are as defined in claim 1;
which is **characterized by** subjecting a compound of formula (I-D) described in claim 5, to reduction.

**20.** A pharmaceutical composition which comprises a pyrimidine *compound* of *formula* (I-D) described in claim 5 or a non-toxic salt thereof, as active ingredient, and a pharmaceutically acceptable carrier.

**21.** Elastase inhibitor which comprises a pyrimidine compound of formula (I-D) described in claim 5 or a non-toxic salt thereof, as active ingredient, and a pharmaceutically acceptable carrier.

**22.** Use of a pyrimidine compound of formula (I-D) described in claim 5 or a non-toxic salt thereof in manufacture of a medicament for the treatment and/or prevention of a disease induced by an abnormal enhancement of degradation of elastin, collagen fiber and / or proteoglycans by elastase.

**23.** Use of a pyrimidine compound of formula (I-D) described in claim 5 or a non-toxic salt thereof in manufacture of a medicament for the treatment and/or prevention of a disease of claim 22, in which a disease is selected from pulmonary emphysema, chronic obstructive pulmonary disease, rheumatoid arthritis, arteriosclerosis, adult respiratory distress syndrome, glomerular nephritis, myocardial infarction, ulcerative colitis and gingivitis.

## INTERNATIONAL SEARCH REPORT

International application No.
PCT/JP00/06601

A. CLASSIFICATION OF SUBJECT MATTER
Int.Cl$^7$ C07D239/36, C07D405/06, C07D413/12, A61K31/505
A61K31/506, A61P11/00, A61P9/00, A61P1/02

According to International Patent Classification (IPC) or to both national classification and IPC

B. FIELDS SEARCHED

Minimum documentation searched (classification system followed by classification symbols)
Int.Cl$^7$ C07D239/36, C07D405/06, C07D413/12
A61K31/505, A61K31/506

Documentation searched other than minimum documentation to the extent that such documents are included in the fields searched

Electronic data base consulted during the international search (name of data base and, where practicable, search terms used)
CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | VEALE C.A. et.al. "Nonpeptidic Inhibitors of Human Leukocyte Elastase.5.", Journal of Medicinal Chemistry; Vol.38(No.1) pp.98-108 (1995) | 1-22 |
| A | WO, 98/24806, A2 (CORTECH Inc.), 11 June, 1998 (11.06.98)<br>& US, 5801148, A & US, 5807829, A<br>& US, 5861380, A & US, 5998379, A<br>& US, 6001811, A & EP, 954526, A2<br>& AU, 9855894, A | 1-22 |
| A | CUESTA E. et.al. "Synthesis of 2-Substituted 4-Oxo-5-nitropyrimidines from Methyl 3-Ethoxy-2-nitroacrylate and Other Reactions" Journal of Heterocyclic Chemistry; Vol.22 (No.2) pp.337-339 (1985) | 1-17 |
| A | WO, 99/28320, A1 (DAINIPPON PHARMACEUTICAL CO., LTD.), 10 June, 1999 (10.06.99), reference examples 15,16<br>& EP, 1036794, A1 & ZA, 9810490, A<br>& AU, 9912604, A | 1-17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 December, 2000 (14.12.00) | 26 December, 2000 (26.12.00) |
| Name and mailing address of the ISA/<br>Japanese Patent Office<br>Facsimile No. | Authorized officer<br>Telephone No. |

Form PCT/ISA/210 (second sheet) (July 1992)

## INTERNATIONAL SEARCH REPORT

International application No.
PCT/JP00/06601

**Box I Observations where certain claims were found unsearchable (Continuation of item 1 of first sheet)**

This international search report has not been established in respect of certain claims under Article 17(2)(a) for the following reasons:

1. ☒ Claims Nos.: 23
because they relate to subject matter not required to be searched by this Authority, namely:
The invention of claim 23 falls under the category of "methods for treatment of the human body by surgery or therapy" as provided for in Rule 39.1(iv) of the Regulations under the PCT.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)